# EUROPÄISCHE PATENTSCHRIFT

(11) **EP 1 565 163 B1**
(45) Veröffentlichungstag und Bekanntmachung des Hinweises auf die Patenterteilung: **01.02.2012**
(21) Anmeldenummer: 03785529.3
(22) Anmeldetag: 25.11.2003
(51) Int. Cl.: A61K 9/107

(54) **EMULSIONSARTIGE WASSERLÖSLICHE KONZENTRATE**
EMULSIVE WATER-SOLUBLE CONCENTRATES
CONCENTRES HYDROSOLUBLES DU TYPE EMULSION

(30) Priorität: 27.11.2002 DE 10255195
(43) Veröffentlichungstag der Anmeldung: 24.08.2005
(73) Patentinhaber: Lipoid GmbH, 67065 Ludwigshafen (DE)
(72) Erfinder: WAJDA, Rudi, 68259 Mannheim (DE)
(74) Vertreter: Schmid, Rudolf
(86) Internationale Anmeldenummer: PCT/DE2003/003887
(87) Internationale Veröffentlichungsnummer: WO 2004/047791

(56) Entgegenhaltungen:
- DE-A- 19 520 659
- DE-A- 19 940 227
- US-A- 5 004 611

## Beschreibung

Die vorliegende Erfindung betrifft emulsionsartige transparente bis durchscheinende wasserlösliche Konzentrate mit den Merkmalen des Anspruchs 1 sowie deren Verwendung für kosmetische, pharmazeutische oder diätetische Zwecke.

Lipophile oder fettartige Substanzen im Pharma-, Kosmetik- und Diätetikbereich müssen üblicherweise in eine problemlos anwendbare Form überführt werden. Eine an sich problemlos anwendbare Form stellt die klassische Emulsion dar, bei der Emulgatoren die Grenzflächenspannung an der Grenzfläche der Fetttröpfchen reduzieren und so für eine feine stabile Verteilung von Fett in Wasser sorgen. Je nach Anwendungsgebiet ergeben sich Emulsionen von cremeartiger bis milchiger Konsistenz, wobei Emulgatorenmengen von 0,5 % -10 % eingesetzt werden. Die Teilchengröße der Fetttröpfchen einer herkömmlichen Emulsion ist von vielen Faktoren abhängig, wie Fett, Emulgator, Energieeintrag und liegt normalerweise im dreistelligen nm-Bereich (100 - 1000 nm)..

Spezielle Fragestellungen und Anwendungen verlangen jedoch transparente bis durchscheinende Produkte mit Tröpfchengrößen im einstelligen bis zweistelligen nm-Bereich (5 - 100 nm). Solche Formulierungen sind nur noch mit Hilfe micellarer Lösungsvermittlung darstellbar. In Form von Mischmicellen werden lipophile Stoffe zusammen mit einem geeigneten Lösungsvermittler (Emulgator) und Coemulgator zu transparenten Formulierungen solubilisiert.
Sowohl in der Kosmetik als auch in der Dermatologie werden optisch ästhetische Produkte benötigt, die schwer wasserlösliche Substanzen teilweise in hohen Konzentrationen enthalten. Neben der äußeren Erscheinung müssen solche Produkte auch eine hervorragende physikalische Stabilität aufweisen. Beispiele sind transparente Ölbäder in der Kosmetik und Dermatologie mit Triglyceriden, Paraffinölen und etherischen Ölen als fettartigen Komponenten.

Parfümöle enthalten ebenfalls überwiegend fettartige Duftstoffe in klar transparenter Form gelöst. Schwer wasserlösliche Pharmazeutika werden bei oraler und parenteraler Applikation ebenfalls zu transparenten Formulierungen verarbeitet.

Ziel eines topisch, oral oder parenteral anzuwendenden Produktes ist es, den lipophilen Wirkstoff anzuwenden. Emulgatoren bzw. Co-emulgatoren stellen unerwünschte aber bis dato technologisch notwendige Hilfsstoffe dar.

Stand der Technik ist, dass die Solubilisierung fettartiger Substanzen zu transparenten Systemen in den oben geschilderten Anwendungsbereichen ausschließlich mit ethoxilierten Tensiden bzw. Tensiden, die einen hohen HLB-Wert besitzen und/oder Alkoholen bewerkstelligt werden kann. Diese Lösungsvermittler besitzen jedoch erhebliche Nachteile:
- die leicht flüchtigen Alkohole - VOC- (Ethanol, Isopropanol) sind starke Zellgifte und
- sind zum Schutz der Erdatmosphäre schon in zahlreichen Formulierungen unerwünscht.
- Emulgatoren müssen zur Solubilisierung in großen Mengen eingesetzt werden, so dass zur Anwendung des eigentlichen Wirkstoffes immer erhebliche Mengen Hilfsstoffe mitgeschleppt werden müssen. Um auf herkömmliche Art beispielsweise 1 g Lavendelöl zu solubilisieren werden 5 g PEG-40 hydriertes Rizinusöl in einer 25 % (w/w) ethanolischen Lösung benöligt. Oder um auf herkömmlich Art beispielsweise 20 g Vitamin E-acetat zu solubilisieren werden 44 q PEG-36 Rizinusöl und 25 g Propylenglykol in einer wässrigen Lösung benötigt. Und zum Solubilisieren der lipophilen Vitamine A, E und D auf herkömmliche Art benötigt man beispielsweise ca. den 10-fachen Überschuss eines Gemisches aus Glycocholsäure und Phosphatidylcholin (cernevit).

In der DE 198 59 427 A1 wird die Herstellung von micellar gelösten fettartigen Substanzen zu transparenten Systemen in Form von Mikroemulsionen beschrieben. Als Emulgatör-/Coemulgatorsystem wird dabei ausschließlich ein Gemisch aus Lecithin und ethoxylierten Emulgatoren, Lecithin/Emulgatoren mit hohem HLB-Wert oder Lecithin/leicht flüchtige Alkohole verwendet. Nachteilig bei dieser Stand der Technik ist es, daß die beschriebenen Emulsionen nicht transparent werden, sondern opaque, milchig bleiben.

DE 199 22 193 beschreibt die Herstellung einer klassischen milchigen Fettemulsion aus hydriertem Lecithin, etherischen Ölen und Wasser und die Herstellung optisch transparenter Konzentrate.

DE 199 40 227 A1 offenbart Phospholipidgel, das durch Zusatz eines mehrwertigen Alkohols oder eines Zuckers stabilisiert wird. Die Konzentration des Alkohols bzw. Zuckers liegt zwischen 0,5 - 40%. Ziel von DE 199 40 227 A1 ist die Stabilisierung eins Phospholipid-**Gels**, das bestimmte Pharmazeutika enthalten kann, gegenüber Verflüssigung. Dies wird durch Verwendung von Polyolen oder Kohlenhydraten erreicht. DE 199 40 227 A1 gibt keinen Hinweis auf Strukturen wie Emulsionen. Aus DE 199 40 227 A1 kann ein Verhältnis Wirkstoff (Aciclovir) zu Phospholipid von 1:4,7 bis 1:5, 5 entnommen werden. Diese Verhältnisse sind typisch für liposomale Zubereitungen. DE 199 40 227 A1 gibt keinerlei Hinweis auf Transparenz und/oder Teilchengröße. Das Phospholipidgel wird gemäß DE 199 40 227 A1 durch Mischen hergestellt.

DE 195 20 659 A1 offenbart eine pharmazeutische Zubereitung zur Therapie, wobei diese Zubereitung neben dem Wirkstoff, 5-35 % eines Phospholipids, 15 50 % eines Alkohols (mehrwertiger Alkohol) und 79 - 0 % Wasser..." enthält. Nach den angegebenen Viskositäten, betrifft DE 195 20 659 A1 offensichtlich Gele. Die Gewichtsverhältnisse zwischen dem Wirkstoff Aciclovir und Phospholipiden lassen auch den Schluss auf eine gelartige liposomale Zubereitung zu. Es handelt sich nicht um eine Emulsion und DE 195 20 659 A1 fehlt jeglicher Hinweis auf Transparenz und Teilchengröße. Die pharmazeutische Zubereitung in DE 195 20 659 A1 wird lediglich durch Mischen hergestellt und funktioniert offensichtlich nur mit Mischungen aus Glycerin und Propandiol, nicht mit Glycerin alleine.

US 5 004 611 offenbart Pro-Liposome Compositions, welche Lecithin und Propylenglykol (35-55 %) enthalten. US 5 004 611 offenbart eine liposomale Zubereitung bzw. einen Vorläufer zur Herstellung einer liposomalen Zubereitung, in US 5 004 611 auch als Pro-liposome bezeichnet. US 5 004 611 offenbart den Einsatz von Propylenglykol, nämlich 2-wertige Alkohole.

EP 0 700 679 A1 *beschreibt eine stabile liposomale injizierbare Formulierung für lipophile*, *schwerlösliche Arzneistoffe* in Form von Liposomen, bei der *der Hochdruckhomogenisator standardmäβig rum Einsatz kommt.* Die Liposome der EP 0 700 679 A1 werden stabilisiert durch den Einbau von kurzkettigen Fettsäuren, bzw. deren Salze. Weiterhin werden diese Liposome während der Herstellung im wässrigen Milieu mit Kryoprotektoren (z.B. Kohlenhydrate) versetzt, um deren Struktur auch im trockenen Zustand zu gewährleisten. Ferner werden die Liposome der EP 0 700 679 A1 mit dem Wirkstoff, den Salzen der kurzkettigen Fettsäure und dem Kryoprotektor im wässrigen Milieu mehrfach hochdruckhomogenisiert, um kleinere Liposome zu erhalten.

EP 0 700 679 A1 beschreibt die Herstellung von Liposome, die sich in Aufbau und Struktur grundsätzlich von Emulsionen unterscheiden. Gemäß EP 0 700 679 A1 werden lipophile Wirkstoffe in die Liposomenmembran eingebaut, was normalerweise nur mit einem großen Überschuss von Phospholipiden zu Wirkstoff möglich ist, nämlich nach EP 0 700 679 A1 Wirkstoff/Phospholipid = 1:20 bis 1:200.

Gemäß EP 0 700 679 A1 werden zur Stabilisierung der Liposomenmembran Salze kurzkettige Fettsäuren verwendet. Diese werden in die Liposomenmembran eingebaut und sind bei neutralem pH überwiegend negativ geladen. Diese negative Ladung auf der Oberfläche der Liposomenmembran verhindert die Fusion der Liposome zu größeren Aggregaten und bewahrt die einmal erzeugte Teilchengröße. Gemäβ EP 0 700 679 A1 wird der wässrigen Iaposomendispersion ein Kryoprotektor zugesetzt. Dieser hat die Aufgabe die Liposomen im trockenen Zustand nach Gefriertrocknung zu stabilisierten.

Der vorliegenden Erfindung liegt nun die Aufgabe zugrunde, schwer wasserlösliche Substanzen zu transparenten oder durchscheinenden micellaren wasserlöslichen Konzentraten zu lösen und diese Konzentrate zu verwenden.

Diese Aufgabe wird erfindungagemäß mit micellaren wasserlöslichen Konzentraten mit den Merkmalen gemäß Anspruch 1 und der Verwendung dieser emulsionsartigen wasserlöslichen Konzentrate mit den Merkmalen gemäß Anspruch 10 gelöst. Im Gegensatz zu bisherigen micellaren Systemen wird gemäß der Erfindung mit einer speziellen Matrix der Vorteil erreicht, dass völlig auf die Verwendung eines zweiten Emulgators und auch auf ein- oder zweiwertige Alkohole verzichtet werden kann. Überraschend vorteilhaft ergibt sich mit der Erfindung weiterhin, dass dies in Bezug auf den Fettkörper mit einem Unterschuss an Phospholipiden möglich ist, also im Gegensatz steht zu den bisherigen konventionellen Möglichkeiten, Lösungsvermittlung zu erreichen.

Ethoxilierte Tenside oder andere starke Lösungsvermittler können mit der Erfindung durch ein System aus natürlichen Substanzen (Lecithine/Phospholipide/Polyole) ersetzt werden, die gesundheitlich unbedenklich und sicher für die Umwelt sind.

Zur Solubilisierung werden mit der Erfindung lediglich 5 - 100 % der Masse des Fettkörpers als Lösungsvermittler (Lecithin/Phospholipid) benötigt. Bei bisherigen Verfahren ist das Verhältnis umgekehrt.

Gemäß der Erfindung kann auf leicht flüchtige organische Lösungsmittel (Ethanol, Isopropanol, u.ä.) verzichtet werden.

Mit Hilfe eines einstufigen Herstellverfahrens entstehen gemäß der Erfindung emulsionsartige Lösungskonzentrate, die nach Verdünnen mit Wasser opak bis transparente, feindisperse Emulsionen ergeben, wie Sie unter Umgehung des emulsionsartigen Konzentrates nicht oder nur wesentlich schwieriger herzustellen wären.

Diese neuartigen Konzentrate können am besten mit Hilfe von Hochdruckhomogenisatoren hergestellt werden.

Rotor-Stator Mischer ergeben weniger transparente Konzentrate. Der Nachteil aus DE 198 59 427, dass durch Hochdruckhomogenisierung Metallrieb auftritt, wird beseitigt, indem zum Homogenisieren in entsprechenden Geräten Keramik eingesetzt wird.

Die erfindungsgemäßen Konzentrate können direkt als Produkt verwendet werden für beispielsweise medizinische oder kosmetische Ölbäder, Mundwässer, Parfümöle, Getränke oder Nahrungsergänzungsmittel oder aber durch Verdünnen mit Wasser oder anderen wasserhaltigen Lösungen (z. B. Säfte) in feindisperse, transparent - opake O/W - Emulsionen (Nanoemulsionen) mit sehr kleinen Teilchengrößenverteilungen im zwei- bis dreistelligen nm-Bereich überführt werden. Bedingt durch die sehr gute Wasserlöslichkeit, können diese transparenten emulsionsartigen Konzentrate problemlos in kosmetische Mittel (Gele, Cremes, Lotionen u.a.), pharmazeutische oder diätetische Produkte eingearbeitet werden.

Die Herstellung eines emulsionsartigen Lösungskonzentrates aus Lecithinen und/oder Phospholipiden, Lipiden und hochkonzentrierten Lösungen von Polyolen oder Kohlenhydraten erfolgt nach folgenden Gesichtspunkten:
- Das Verhältnis Lecithin/Phospholipid zu fettartiger Substanz sollte so gewählt werden, dass ein transparentes Konzentrat entsteht, das sich problemlos mit Wasser verdünnen lässt und
- der Wasseranteil der Polyol- oder Kohlenhydratlösungen sollte so gewählt werden, dass nicht nur ein transparentes Konzentrat entsteht, sondern auch - aufgrund des geringen Wasserwertes - ein selbstkonservierendes System, so dass auf Zusatz von synthetischen oder auch natürlichen Konservierungsmitteln verzichtet werden kann.
- Die Verarbeitungstemperatur sollte den verwendeten Lecithinen/Phospholipiden bzw. den zu solubilisierenden Lipiden angepasst. sein. Bei hydrierten Lecithinen/Phospholipiden sind naturgemäß Verarbeitungstemperaturen zwischen 40 und 80 °C nötig bei ungesättigten Lecithinen/Phospholipiden kann bei Raumtemperatur gearbeitet werden, wobei die Lecithine/Phospholipide bevorzugt in die polare Phase, d. h. in die Polyol- bzw. Kohlenhydratlösungen eingearbeitet werden.

Die vorliegende Erfindung soll im folgenden an Hand von bevorzugten Ausführungsbeispielen dargestellt werden.

### Beispiel 1:

5 g einer Phospholipidfraktion aus Soja mit einem PC-Gehalt von 70 % werden in 75 g 86 %-igem Glycerin unter Rühren dispergiert. 20 g Vitamin E-acetat zugeben und unter Weiterrühren verteilen. Diese grobdisperse inhomogene Mischung mittels Hochdruckhomogenisator homogenisieren. Es entsteht eine transparente emulsionsartige Lösung hoher Viskosität.

### Beispiel 2:

5 g einer Phospholipidfraktion aus Soja mit einem PC-Gehalt von 70 % werden in 75 g einer 70 %-igen Fructoselösung unter Rühren dispergiert. Nach Zugaben von 20 g eines mittelkettigen Triglycerides und Hochdruckhomogenisieren dieser Mischung, entsteht eine transparente emulsionsartige Lösung honigartiger Viskosität.

## Patentansprüche

1. Wasserlösliche Emulsionskonzentrate von fettartigen Substanzen hergestellt mit den folgenden Schritten:
Kombinieren von Lecithinen und hochkonzentrierten wässrigen Lösungen aus höher als zweiwertigen Alkoholen mit Kettenlängen von C3-C6 oder Kohlenhydraten, wobei die Konzentration der Lösungen aus höher als zweiwertigen Alkoholen mit Kettenlängen von C3-C6 bzw. Kohlenhydraten zwischen 30 Gew.-% und 99 Gew.-% ist,
Dispergieren der Kombination mittels Rühren,
Zugeben der fettartigen Substanzen, wobei das Verhältnis Lecithin:fettartigen Substanzen zwischen 1:1 und 1:12 und die Konzentration der Mischung Lecithin/fettartigen Substanzen in den hochkonzentrierten wässrigen Lösungen aus höher als zweiwertigen Alkoholen mit Kettenlängen von C3-C6 oder Kohlenhydraten zwischen 10 Gew.-% und 90 Gew.-% ist, Rühren der Kombination und der fettartigen Substanzen und Homogenisieren unter Hochdruck.

2. Wasserlösliche Emulsionskonzentrate nach Anspruch 1, **dadurch gekennzeichnet, dass** die verwendeten Lecithine entweder ungesättigt, hydriert, hydrolysiert oder hydroxyliert sind, wobei die Lecithine aus Soja, Raps, Fisch, Milch oder Ei gewinnbar sind und die Lecithinfraktionen aus entölten Fraktionen mit Phosphatidylcholingehalten zwischen 10 Gew.-% und 100 Gew.-% gebildet sind.

3. Wasserlösliche Emulsionskonzentrate nach Anspruch 1, **dadurch gekennzeichnet, dass** Lecithine mindestens 50 Gew. -% acetonunlösliche Substanzen enthalten, überwiegend aus der Gruppe der polaren, fettartigen Substanzen, wie Glycerinphosphatide, Sphingophosphatide, Sphingoglykolipide, Glyceringlykolipide oder Aminolipide.

4. Wasserlösliche Emulsionskonzentrate nach Anspruch 1, **dadurch gekennzeichnet, dass** als Kohlenhydrate Monosaccharide, Disaccharide, Maltitol und Maltodextrine verwendet werden.

5. Wasserlösliche Emulsionskonzentrate nach Anspruch 1, **dadurch gekennzeichnet, dass** keine ethoxylierte oder andere synthetische Tenside verwendet werden.

6. Wasserlösliche Emulsionskonzentrate nach Anspruch 1, **dadurch gekennzeichnet, dass** keine Konservierungsmittel verwendet werden.

7. Wasserlösliche Emulsionskonzentrate nach Anspruch 1, **dadurch gekennzeichnet, dass** bevorzugt Substanzen aus der Gruppe der fettartigen Substanzen (z. B. Fettsäuren, Wachse, Wachsester, Paraffine, Fettalkohole, Fettaldehyde, Glyceride usw. ), Isoprenoide (Terpene und Steroide : z. B. A-Vitamine, E-Vitamine, D-Vitamine, K-Vitamine, Bisabolol, Menthol, Glucocorticoide, etherische Öle, Cholesterin, Sitosterole, Coenzym Q 10, usw.), der polaren fettartigen Substanzen (Ceramide, Sphingolipide, Glykolipide, usw. ) sowie der öllöslichen UV-A und UV-B Filter und Siliconöle verwendet werden.

8. Verwendung der wasserlöslichen Emulsionskonzentrate gemäß mindestens einem der vorhergehenden Ansprüche für kosmetische, pharmazeutische oder diätetische Zwecke.

9. Verwendung gemäß Anspruch 8, **gekennzeichnet durch** topische, orale oder parenterale Anwendung.

## Claims

1. Water soluble emulsion concentrate of fatty substances made by the following steps:
Combining of lecithin and highly concentrated aequous solutions from higher than bivalent alcohols with chain lengths of C3-C6 or carbohydrates, wherein the concentration of solutions from higher than bivalent alcohols with chain lengths of C3-C6 or carbohydrates is between 30 % by weight and 99 % by weight,
Dispersing of the combination by steering,
Adding of the fatty substances, with the ratio of lecithin:fatty substances being between 1:1 and 1:12 and the concentration of the mixture of lecithin:fatty substances in the highly concentrated aequous solutions from higher than bivalent alcohols with chain length of C3-C6 or carbohydrates being between 10 % by weight and 90 % by weight,
Steering of the combination and the fatty substances and homogenizing under high pressure.

2. Water-soluble emulsion-concentrate according to claim 1, wherein the applied lecithins are either unsaturated, hydrogenated or hydroxylated, with the lecithins being extracted from soya, rape, fish, milk or egg and fractions of the lecithin are formed oil-free with parts of phosphatidylcholine from 10 % by weight to 100 % by weight.

3. Water-soluble emulsion-concentrate according to claim 1, wherein the lecithin contains at least 50 % by weight of substances not soluble in acetone, and is mostly selected from the group of polar fatlike substances, such as glycerinphosphatides, sphingophosphatides, spingoglycolipids, glyceringlycolipids and aminolipids.

4. Water-soluble emulsion-concentrate according to claim 1, wherein the carbohydrate is selected from the group comprising monosaccharides, disaccharides, maltitol and maltodextrins.

5. Water-soluble emulsion-concentrate according to claim 1, wherein no ethoxylated surfactants or other synthetic surfactants are used.

6. Water-soluble emulsion-concentrate according to claim 1, wherein no preservatives are used.

7. Water-soluble emulsion-concentrate according to claim 1, wherein preferably substances of the group of the fatlike substances (e.g. fatlike acids, waxes, wax esters, paraffins, fatlike alcohols, fatlike aldehydes, glycerides, etc.) isoprenoides (terpenes and steroids such as vitamin-A, vitamin-E, vitamin-D, vitamin-K, bisabolol, menthol, glucocorticoids, essential oils, cholesterol, sitosterols, coenzyme Q 10, etc.), of the polar fatlike substances (ceramides, sphingolipids, and glycolipids, etc.) as well as the oil soluble UV-A and UV-B filters and silicon oils are applied.

8. Application of the water-soluble emulsion-concentrate according to at least one of the preceding claims for cosmetic, pharmaceutical or dietetic applications.

9. Application of the water-soluble emulsion-concentrate according to claim 8, **characterized by** topical, oral or parenteral application.

## Revendications

1. Concentrés d'émulsion hydrosolubles des matières à caractère gras produit selon des pas suivants:
Combiner des lécithines et des solutions aqueuses à concentration élevée des alcools plus que bivalents à longue chaine de C3-C6 ou hydrate de carbone, la concentration des solutions des alcools plus que bivalents à longue chaine de C3-C6 ou hydrate de carbone étant entre 30 poids-% et 99 poids-%,
Disperger la combinaison par agitation,
Ajouter les matières à caractère gras, le rapport lécithine:matières à caractère gras étant entre 1:1 et 1:12 et la concentration des mélanges lécithine:matières à caractère gras dans les solutions aqueuses à concentration elevée des alcools plus que bivalents à longue chaine de C3-C6 ou hydrate de carbone étant entre 10 poids-% et 90 poids-%,
Agiter la combinaison et les matières à caractère gras et homogénéiser sous haute pression.

2. Concentrés d'émulsion hydrosolubles selon revendication 1, **caracterisé en ce que** les lécithines utilisées soient ou insaturées, hydrogenées, hydrolisées ou hydroxylées, les lécithines étant gagnables de soja, colza, poisson, lait ou oeuf et les fractions de lécithine sont formées des fractions sans huiles avec un teneur de phosphatidylcholin entre 10 poids-% et 100 poids-%.

3. Concentrés d'émulsion hydrosolubles selon revendication 1, **caracterisé en ce que** les lécithines contiennent au moins 50 poids-% des matières insolubles en acétone en majorité du groupe des matières polaires à caractère gras, comme phosphatides à glycérine, sphingophosphatides, sphingoglycolipide, glicérine à glycolipide ou aminolipide.

4. Concentrés d'émulsion hydrosolubles selon revendication 1, **caracterisé en ce que** des monosaccharides, disaccharides, maltitoles et maltodextrines sont utilisés comme hydrates de carbone.

5. Concentrés d'émulsion hydrosolubles selon revendication 1, **caracterisé en ce que** aucun tenside ethoxylé ou synthetic est utilisé.

6. Concentrés d'émulsion hydrosolubles selon revendication 1, **caracterisé en ce que** aucun agent conservateur est utilisé.

7. Concentrés d'émulsion hydrosolubles selon revendication 1, **caracterisé en ce que** en priorité sont utilisés des matières du groupe des matières à caractère gras (par exemple des acides gras, cire, ester de cire, paraffine, des alcools gras, des aldéhydes gras, des glycérides, etc.), des isoprénoides (des terpènes et des stéréoides: par exemple des vitamines-A, des vitamines-E, des vitamines-D, des vitamines-K, bisabolol, menthol, des glucocorticoides, des huiles ethériques, du cholestérol, du sitostérol, du coenzyme Q-10, etc.), les matières polaires à caractère gras (des céramides, des sphingolipides, des glycolipides, etc.) ainsi que les filtres UV-A et UV-B solubles en huile.

8. Application des concentrés d'émulsion hydrosolubles selon au moins une des revendications precedents pour des fins cosmétiques, pharmaceutiques ou diététiques.

9. Application selon revendication 8, **caracterisé par** application topique, orale ou parentérale.
